(19) Europäisches Patentamt / European Patent Office / Office européen des brevets

(11) **EP 2 564 873 A1**

## (12) EUROPEAN PATENT APPLICATION
published in accordance with Art. 153(4) EPC

(43) Date of publication:
06.03.2013 Bulletin 2013/10

(51) Int Cl.:
*A61K 47/10* (2006.01)    *A61K 9/70* (2006.01)
*A61K 31/137* (2006.01)    *A61K 31/138* (2006.01)
*A61K 31/343* (2006.01)    *A61K 31/407* (2006.01)
*A61K 31/465* (2006.01)    *A61K 31/517* (2006.01)

(21) Application number: 11775064.6

(22) Date of filing: 27.04.2011

(86) International application number:
PCT/JP2011/060290

(87) International publication number:
WO 2011/136283 (03.11.2011 Gazette 2011/44)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 28.04.2010 JP 2010104125

(71) Applicant: Hisamitsu Pharmaceutical Co., Inc.
Tosu-shi, Saga 841-0017 (JP)

(72) Inventors:
• KUMA Hidekazu
Tsukuba-shi
Ibaraki 305-0856 (JP)
• ATARASHI Kenji
Tsukuba-shi
Ibaraki 305-0856 (JP)
• SUZUKI Kazuhiro
Tsukuba-shi
Ibaraki 305-0856 (JP)
• AIDA Kazunosuke
Tsukuba-shi
Ibaraki 305-0856 (JP)

(74) Representative: Grünecker, Kinkeldey,
Stockmair & Schwanhäusser
Leopoldstrasse 4
80802 München (DE)

## (54) SKIN IRRITATION SUPPRESSANT AND TRANSDERMAL PREPARATION

(57) Provided is a skin irritation suppressant for transdermal preparations, having a sufficient reduction effect of skin irritation due to a drug. Also provided is a transdermal preparation comprising the skin irritation suppressant. One embodiment of the invention is a skin irritation suppressant for suppressing the skin irritation due to a drug and a pharmaceutical ingredient to be used in a transdermal preparation other than the drug, the skin irritation suppressant comprising a sterol compound selected from the group consisting of cholesterol, cholesterol derivatives and cholesterol analogs, and the drug is one or more basic drugs selected from the group consisting of tolterodine, asenapine, bisoprolol, risperidone, nicotine and citalopram, and their pharmaceutically acceptable salts.

Fig. 3

EP 2 564 873 A1

## Description

## Technical Field

The invention relates to a skin irritation suppressant and a transdermal preparation containing the skin irritation suppressant.

## Background Art

Transdermal preparation, which allows administration of a drug through the skin, has the advantages in comparison with injection or oral preparation that a rapid increase in blood level of the drug can be avoided, absorption of the drug can be easily sustained, the hepatic first pass effect can be avoided, the administration can be discontinued when side effects occur, and the like,. In contrast, there has been the case where the drug was administrated by the application of transdermal preparation, and skin irritations such as pruritus, flushing, rash, pain, eczema and dermatitis occurred in the skin to which the preparation is applied.

It is described in Patent Literature 1 that skin irritations due to selective serotonin reuptake inhibitor is reduced by addition of hydroquinone glycoside, pantethine, tranexamic acid or lecithin. In addition, it is described in Patent Literature 2 that titanium oxide and aluminum hydroxide reduce skin irritations due to non-steroidal anti-inflammatory agents.

Although it has been well known that steroids with steroidal backbone generally have anti-inflammatory effects, for example, Patent Literature 3 describes that cholesterol have no anti-inflammatory activity. Meanwhile, it is described in Patent Literature 4 that cholesterol are effective in suppressing skin irritation of a tape-type transdermal preparation (plaster) containing bisphosphonate.

## Citation List

## Patent Literature

Patent Literature 1: Japanese Patent Laid-Open No. 2007-284378
Patent Literature 2: Japanese Patent Laid-Open No. 2007-045738
Patent Literature 3: Japanese Patent Laid-Open No. 1992-501415
Patent Literature 4: WO 2009/075258

## Summary

## Technical Problem

However, in the methods described in Patent Literatures 1 and 2, reduction effect of skin irritation can only be obtained for specific drugs such as the selective serotonin reuptake inhibitor and the non-steroidal anti-inflammatory agent, and there has been a case where the reduction effect of skin irritation are insufficient. Additionally, the effects of the cholesterol in the transdermal preparation described in Patent Literature 3 is limited to the bisphosphonate-containing transdermal preparation, and reduction effect of skin irritation are not confirmed in transdermal preparation containing other drugs.

Therefore, one object of the invention is to provide a skin irritation suppressant for the transdermal preparation having sufficient reduction effect of skin irritation on a wide range of drugs, and a transdermal preparation containing the skin irritation suppressant.

## Solution to Problem

As a result of extensive and intensive studies to solve the problems, the inventors have found that cholesterol which have been considered not to have anti-inflammatory activity exhibits a reduction effect (suppressive effect) of skin irritation due to drugs, thereby having completed the invention.

That is, one embodiment provides a skin irritation suppressant for suppressing skin irritation due to a drug and a pharmaceutical ingredient to be used in a transdermal preparation other than the drug, comprising a sterol compound selected from the group consisting of cholesterol, cholesterol derivatives and cholesterol analogs, wherein the drug is one or more basic drugs selected from the group consisting of tolterodine, asenapine, bisoprolol, risperidone, nicotine and mitalopram, or their pharmaceutically acceptable salts. By the skin irritation suppressant, sufficient reduction effect of skin irritation on a wide range of drugs and/or pharmaceutical ingredients to be used for the transdermal preparation other than the drugs can be obtained.

In some embodiments, the pharmaceutical ingredient to be used in a transdermal preparation other than the drug is a transdermal absorption enhancer, and in case the transdermal absorption enhancer is selected from lauric acid diethanolamide (LADA), propylene glycol monolaurate and sorbitan monolaurate, particularly remarkable reduction effect of skin irritation can be obtained.

Another embodiment provides a transdermal preparation which comprises an effective amount of the skin irritation suppressant, the drug and/or the pharmaceutical ingredient. By the transdermal preparation, sufficient reduction effect of skin irritation can be obtained without affecting the release amount of the drug.

In some embodiments, the transdermal preparation comprises 0.1-30% by mass of the skin irritation suppressant relative to the total amount of the transdermal preparation. In addition, in some further embodiments, the mass ratio of the drug to the skin irritation suppressant in the transdermal preparation is from 30:1 to 1:10. Higher reduction effect of skin irritation can be obtained by such transdermal preparation.

**Advantageous Effects**

There is provided a skin irritation suppressant for the transdermal preparation which exhibits sufficient reduction effect of skin irritation on a wide range of drugs, and a transdermal preparation containing the skin irritation suppressant.

**Brief Description of Drawings**

[Fig. 1] Fig. 1 is a graph showing results of Experiment 4.
[Fig. 2]Fig. 2 is a graph showing results of Experiment 5.
[Fig. 3]Fig. 3 is a graph showing results of Experiment 7.

**Description of Embodiments**

(Skin irritation suppressant)

In one embodiment, the skin irritation suppressant is selected from the group consisting of cholesterol, cholesterol derivatives and cholesterol analogs. Cholesterol is $(3\beta)$-cholest-5-en-3-olcholest-5-en-3$\beta$-ol and known as an essential component in cell membranes of higher animals. Cholesterol derivative means a natural or synthetic cholesterol derivative and is exemplified by an acylcholesterol which is an ester compound in which a fatty acid binds to a part of a hydroxy group. In addition, cholesterol analog means a natural or synthetic cholesterol analog and is exemplified by, for example, plant cell-derived phytosterols such as sitosterol, stigmasterol, fucosterol, spinasterol, campesterol and brassicasterol, and a fungous-derived ergosterol, etc.

All of cholesterol, cholesterol derivatives and cholesterol analogs are classified as steroids, and belongs to a subgroup among them referred to as sterol (steroid alcohol). The skin irritation suppressant can be any one of or a combination of two or more of these sterol compounds.

By the skin irritation suppressant, skin irritation due to a drug, particularly a drug that is likely to cause skin irritation, can be reduced.

Immune reactions of epidermal cells are variously studied as one of the mechanisms for skin irritation of drugs. Epidermal cells play a central role in cutaneous immunity by releasing many proinflammatory substances such as cytokine, chemokine, inflammatory mediator and cell growth factor to the outside of the cells, and by expressing cytokine receptor, adhesion factor and MHC class II on the cells ("Handbook of Cutaneous Immunity" published by CHUGAI IGAKUSHA).

Proinflammatory substances (skin irritation mediator) released by the epidermal cells include interleukin (IL)-1$\alpha$, IL-10, IL-12, IL-18, TNF-$\alpha$, GM-CSF, IL-6, IL-7, IL-15, TGF-$\alpha$, amphiregulin, HB-EGF, bFGF, VEGF, PDGF, SCF, IFN-$\beta$, IFN-$\gamma$, TGF-$\beta$, MIP-3$\alpha$, IP-9, IP-10, Mig, IL-8, GRO$\alpha$, RANTES, MCP-1, TARC, prostaglandin, leukotriene, substance P, reactive oxygen species, nitrogen oxide and the like, and they cover a considerably broad range and intricately interact with each other to modulate immune reaction.

Therefore, in the present specification, "reduction in skin irritation" herein means reduction in production of a so-called skin irritation mediator induced by a drug, such as prostaglandin E2 (PGE2), IL-1$\alpha$, IL-6 and IL-8 by the drug in an *in vitro* test using the epidermal cell, and/or means reduction in skin irritation such as skin erythema and swelling formation *in vivo.* The skin irritation can be evaluated, for example, by calculating the skin Primary Irritation Index (PII) based on each scores for erythema and swelling at the application region in accordance with the evaluation standard of Draize et al. (Reference literature: Draize JH, et al. J Pharmacol Exp Ther. 1944; 82: 377-390).

It should be noted that skin irritation herein means side effects such as pruritus, flushing, rash, pain, eczema and dermatitis which are caused when the transdermal preparation is administered to the topical skin.

In the transdermal preparation, the skin irritation suppressant is added together with one or more basic drugs selected from the group consisting of tolterodine, asenapine, bisoprolol, risperidone, nicotine and mitalopram, and pharmaceutically acceptable salts thereof, and/or a pharmaceutical ingredient to be used in a transdermal preparation other than the drug. Skin irritation due to the drug and/or the pharmaceutical ingredient to be used in a transdermal preparation other than the drug can be significantly reduced by the skin irritation suppressant. All of the drugs are basic drugs which act on receptors. The inventors think that the reduction effects of skin irritation, induced by these basic drugs, of the skin irritation suppressant are caused by the fact that the particular cholesterol compounds suppress the production of inflammatory mediators induced by these basic drugs.

Herein, pharmaceutical ingredient to be used in a transdermal preparation other than drug means a component other than active ingredients (so called "drug") contained in the transdermal preparation and vary on the kind of the transdermal preparation, and generally they include a stabilizing agent, a surfactant, a plasticizer, a lubricant, a solubilizer, a reductant, a buffer, a sweetening agent, a base, a volatilization adjuvant, an absorption enhancer, a synergist, a binder, a suspending agent, a hardener, an antioxidant, a polish, a perfume, a potency enhancer, a coating agent, a prolonging agent, a moistening agent, a moisture regulator, a filler, an algefacient, an bond, a potentiator, a flavor, a coloring agent, a sugar-coating agent, a tonicity agent, a softener, an emulsifier, an adhesive, an adhesion enhancer, a viscosity modifier, an inflammation suppressant, an exothermic agent, a foaming agent, a pH adjuster, a skin protectant, an excipient, a flotation agent, a dispersant, a disintegrant, a disintegration adjuvant, a fragrance, a desiccant, an antiseptic agent, a scavenger, a preservative, a soothing agent, an attractant, a dissolving agent, a dissolving adjuvant, a solvent, a mold release agent, a fluidizer, etc. Among these pharmaceutical ingredients, there are included, as the pharmaceutical ingredients causing skin irritation, a stabilizing agent, a surfactant, a plasticizer, a solubilizer, a reductant, a buffer, a base, an absorption enhancer, a suspending agent, an antioxidant, a perfume, an algefacient, a bond, an adhesive, an adhesion enhancer, an excipient, a fragrance, a desiccant, an antiseptic agent, a preservative, a dissolving agent, a dissolving adjuvant, a solvent, etc. Above all, the skin irritation due to the transdermal absorption enhancer selected from the absorption enhancer, particularly lauric acid diethanolamide (LADA), propylene glycol monolaurate and sorbitan monolaurate can be significantly reduced by the skin irritation suppressant. The inventors think that the reason for this is because the particular sterol compounds may probably suppress the production of the inflammatory mediators due to these pharmaceutical ingredients.

(Transdermal preparation)

The transdermal preparation in another embodiment comprises an effective amount of a skin irritation suppressant, a drug and/or a pharmaceutical ingredient to be used in a transdermal preparation other than the drug. The content of the skin irritation suppressant in the transdermal preparation may be an amount capable of obtaining effects (effective amount) of reducing the skin irritation and vary on the kind of the transdermal preparation, and it may be 0.1-30% by mass, 0.3-10% by mass, and furthermore 0.5-5% by mass relative to the total amount of the transdermal preparation. The skin irritation due to the drug and/or the pharmaceutical ingredient to be used in a transdermal preparation other than the drug can be reduced by addition of the skin irritation suppressant. It should be noted that the total amount of the transdermal preparation herein means the total mass of the drug-containing part. That is, in the case of a dosage form such as an ointment, a cream, a gel, a gelled cream, a liniment and a lotion, the total amount means the total mass, in the case of a dosage form such as a cataplasm, a plaster and a reservoir-type patch, it means the mass of a part other than a backing, and in the case of a dosage form such as a spray and an aerosol, it means the mass of the part other than the container part.

From the viewpoint of reduction in skin irritation, one or more basic drugs selected from the group consisting of tolterodine, asenapine, bisoprolol, risperidone, nicotine and mitalopram, and their pharmaceutically acceptable salts are preferably used as drugs added to the transdermal preparation.

When transdermal preparation comprises a pharmaceutical ingredient to be used in a transdermal preparation other than drug, it may not comprise the basic drug but comprise other drugs or active ingredients, or it may comprise other drugs or active ingredients with the basic drug when they are acceptable from the viewpoint of drug interaction or the like. Other drugs or active ingredients include: antihypertensives such as atenolol, amlodipine and captopril; vasodilators such as isosorbide dinitrate and nitroglycerine; calefacients such as capsaicin, red pepper extract, red pepper powder, red pepper tincture, nonylic acid vanillylamide; non-steroidal antiphlogistic analgetics such as indomethacin, camphor, ketoprofen, methyl salicylate, glycol salicylate, diclofenac sodium, flurbiprofen, felbinac, meloxicam and loxoprofen; hormone drugs such as estradiol, norethisterone and estriol; antihistamines such as ketotifen; anti-Alzheimer drugs such as memantine and donepezil; antidepressants such as sertraline, fluoxetine, paroxetine, citalopram and fluvoxamine; drugs for treatment of gastric ulcer such as teprenone; drugs for treatment of overactive bladder such as oxybutynin and solifenacin; bronchodilators such as tulobuterol; refrigerants such as menthol and mentha oil; drugs for treatment of Parkinson's disease such as pergolide and rotigotine; vitamin preparations such as retinoid, etc. It is assumed that the skin irritation due to these other drugs and active ingredients may also be reduced by addition of the skin irritation suppressant.

The content of the drugs in the transdermal preparation may be an effective amount for each drug, and it may vary on the kind of the transdermal preparation, 0.1-30% by mass, 0.3-10% by mass, and furthermore 0.5-5% by mass relative to the total amount of the transdermal preparation. In addition, a mass ratio of the drug to the skin irritation suppressant in the transdermal preparation may be from 30:1 to 1:10, from 25:1 to 1:5, from 20:1 to 1:3, and furthermore from 20:1 to 1:1. These mass ratios enhance reduction in skin irritation and do not prevent release of the drug.

Although the dosage form of the transdermal preparation is not particularly limited, it may be ointment, cream, gel, gelled cream, liniment, lotion, spray, aerosol, cataplasm, plaster, reservoir-type patch, etc. which have been conventionally used as external preparations. Among them, cataplasm, plaster and reservoir-type patch are particularly preferable.

In one embodiment, the transdermal preparation is a cataplasm. The cataplasm includes a backing and a drug layer laminated on at least one surface of the backing. The drug layer includes a drug, a skin irritation suppressant and a base. In consideration of temporal stability, release performance, transdermal absorbability, skin stability, the base is preferably a hydrophilic base comprising a water-soluble polymer, a polyhydric alcohol and water.

As the water-soluble polymer, one of or two or more of compounds are optionally selected from gelatin, casein, pullulan, dextran, alginate sodium, soluble starch, carboxy starch, dextrin, carboxymethyl cellulose, sodium carboxymethylcellulose, methylcellulose, ethylcellulose, hydroxyethyl cellulose, polyvinyl alcohol, polyethylene oxide, polyacrylic acid, polyacrylamide, sodium polyacrylate, polyvinyl pyrrolidone, carboxyvinyl polymer, polyvinyl ether, methoxyethylene anhydrous maleic acid copolymer, isobutylene anhydrous maleic acid copolymer, N-vinylacetamide, copolymer of N-vinylacetamide, acrylic acid and acrylate, etc. The content of the water-soluble polymer may be 1-30% by mass, 1-20% by mass, and furthermore 1-15% by mass relative to the total amount of the transdermal preparation. When the content is less than 1% by mass, its viscosity becomes low, and thus shape retention cannot be kept, and when the content is more than 30% by mass, its viscosity becomes high, and thus workability at the time of kneading and application may be reduced.

As the polyhydric alcohol, one of or two or more of compounds are optionally selected from polyethylene glycol, propylene glycol, dipropylene glycol, polypropylene glycol, 1,3-butylene glycol, 1,4-butylene glycol, isobutylene glycol, glycerin, diglycerin, sorbitol, etc. The content of the polyhydric alcohol may be 5-90% by mass, 10-70% by mass, furthermore 20-60% by mass relative to the total amount of the transdermal preparation. When the content is less than 5% by mass, its moisturizing action may be insufficient, and when the content is more than 90% by mass, a solubility of the water-soluble polymer may be affected. The content of water may be 10-90% by mass or 20-80% by mass relative to the total amount of the transdermal preparation. The water-soluble polymer can be dissolved by addition of water, and thus that thickening property, aggregability and shape retention can be brought out.

A cross-linking agent may be added into the drug layer of the cataplasm if necessary. As the cross-linking agent, there can be optionally added one compound of or two or more of compounds selected from polyvalent metal compounds such as aluminum hydroxide, aluminium chloride, calcium hydroxide, calcium chloride, aluminium sulfate, aluminium ammonium sulfate, aluminum potassium sulfate, magnesium aluminometasilicate, and dihydroxyaluminum amino acetate; compounds having at least two or more epoxy groups in a molecule such as ethylene glycol diglycidyl ether, polyethylene glycol diglycidyl ether, propylene glycol diglycidyl ether, polypropylene glycol diglycidyl ether, polytetramethylene glycol diglycidyl ether, glycerol polyglycidyl ether, polyglycerol polyglycidyl ether, sorbitol polyglycidyl ether, sorbitan polyglycidyl ether, trimethylolpropane polyglycidyl ether, pentaerythritol polyglycidyl ether, resorcin diglycidyl ether, neopentylglycol diglycidyl ether, and 1,6-hexanediol diglycidyl ether.

The drug layer of the cataplasm may optionally contain not only the cross-linking agent but also one of or two or more of compounds selected from fillers such as kaolin, zinc oxide, titanium dioxide, talc, bentonite and synthetic aluminum silicate; antiseptic agents such as thymol, methylparaben and ethylparaben; antioxidants such as ascorbic acid, stearic acid ester, dibutyl hydroxytoluene, butylated hydroxyanisole, gallic acid ester, vitamin E, vitamin E acetic acid ester and disodium edetate; ultraviolet absorbers such as 2-hydroxy-4-methoxybenzophenone, ethyl p-aminobenzoate, 2-(2-hydroxy-5-methylphenyl) benzotriazole, glycol salicylate, methyl salicylate and phenyl salicylate; and emulsifiers such as sorbitan fatty acid ester, glycerine fatty acid ester, deca glycerine fatty acid ester, polyoxyethylene sorbitan fatty acid ester, polyethylene glycol fatty acid ester and polyoxyethylene alkyl ether.

As a backing of the cataplasm, materials which do not affect release of the drug can be used. That is, a backing which does not interact with the drug and to which the drug adsorb is preferable, and available materials include a film and a sheet of polyethylene, polypropylene, polyvinyl chloride, polyester, nylon and polyurethane, etc., and a porous material, a foam, a cloth or a non-woven fabric, and their laminated articles, etc. More specifically, Sand matte PET (Teijin Dupont Films Japan Limited) and Scotchpak (Trademark) 9732 (3M Company) or the like can be used. In the drug layer of the cataplasm, surface on the opposite side of a surface contacting a backing may be provided with a release liner which is peeled off for use before being applied to an affected part. As the release liner, polyethylene, polypropylene, polyester, polyethylene terephthalate, or their materials subjected to mold release treatment with a silicone, a release paper, etc. can be used.

Next, a manufacturing method of the cataplasm will be explained. A water-soluble polymer is mixed with a polyhydric alcohol and water, dispersed and dissolved therein and a uniform kneaded material of the base is obtained. If necessary, an antioxidant, an ultraviolet absorber, an emulsifier, an antiseptic agent, etc. are added to the base. Subsequently, the drug and the skin irritation suppressant are added to the base, and uniformly dispersed to directly spread on the backing, or are spread on the paper or film once subjected to release treatment and then are transferred to the backing by pressure bonding. Next, the surface on the opposite side of the surface contacting the backing in the drug layer is covered with the release liner, and by being cut into the appropriate size, the cataplasm was obtained. It should be noted that the blending order of each component in the manufacturing method is nothing but one example, and is not limiting.

In another embodiment, the transdermal preparation is a plaster. The plaster is provided with a backing and a drug layer which is laminated on at least one surface of the backing. The drug layer includes the drug, the skin irritation suppressant, and an adhesive base. The adhesive base is exemplified by an acrylic adhesive base, a rubber adhesive base, a silicone adhesive base, etc.

As the acrylic adhesive base, a single polymer or a copolymer of a (meth)acrylic acid alkyl ester having an alkyl group of 4-18 carbon atoms, or a copolymer of the (meth)acrylic acid alkyl ester and another functional monomer is preferably used. It should be noted that the (meth)acryl means acryl or methacryl.

The rubber adhesive base is exemplified by a natural rubber, a synthetic isoprene rubber, polyisobutylene, polyvinyl ether, polyurethane, polyisoprene, polybutadiene, styrene-butadiene copolymer, styrene-isoprene copolymer, styrene-isoprene-styrene (SIS) block copolymer, etc.

As the silicone adhesive base, those comprising polyorganosiloxane or polydimethylsiloxane as major ingredient are used.

The drug layer of the plaster may contain a tackifying agent together with the adhesive base. The tackifying agent is exemplified by rosin and a rosin-based tackifying agent such as a hydrogenated, disproportioned, polymerized or esterized rosin derivative; a terpene resin such as $\alpha$-pinene and $\beta$-pinene; a terpene-phenol resin; an aliphatic, aromatic, alicyclic or copolymeric petroleum resin; an alkyl-phenyl resin; a xylene resin, etc.

Furthermore, the drug layer of the plaster may contain a softener. The softener plasticizes and softens the adhesive base to thereby maintain appropriate adherability to the skin. The softener is exemplified by higher aliphatic acid esters such as polybutene, polyisobutylene, liquid paraffin and isopropyl myristate; silicone oil; and vegetable oils such as almond oil, olive oil, camellia oil, persic oil and peanut oil.

It is preferable that the backing of the plaster does not affect release of the drug, for which stretch or non-stretch backings are used, and a film and a sheet of polyethylene, polypropylene, polybutadiene, ethylene-vinyl acetate copolymer, polyvinyl chloride, polyester, nylon, polyurethane, etc., and their layered products, a porous material, a foam, a cloth and a non-woven fabric, and their laminated articles, etc. can be used. In the drug layer of the plaster, surface on the opposite side of the surface contacting the backing may be provided with a release liner which is peeled off for use before being applied to an affected part. As the release liner, polyethylene, polypropylene, polyester, polyethylene terephthalate, or their materials subjected to mold release treatment with a silicone, a release paper, etc. can be used.

Subsequently, a manufacturing method of the plaster will be explained. In the case of the plaster using an acrylic adhesive base, the adhesive base, the drug and the skin irritation suppressant are dissolved or dispersed in a solvent, and a resulting solution or dispersion liquid is directly coated on the surface of the backing and dried and a drug layer with a thickness of 30-200 $\mu$m is formed, or the solution or dispersion liquid is coated on a paper or film subjected to release treatment and an attaching layer obtained after drying is transferred to the backing by pressure bonding. Next, the surface on the opposite side of the surface contacting the backing in the drug layer is covered with a release liner, and the product is cut into an appropriate size and the plaster is obtained. It should be noted that the blending order of each component in the manufacturing method is nothing but one example, and is not limiting. A solvent to be used in this manufacturing method is not particularly limited as long as it is an organic solvent compatible with all the blending components such as the adhesive base and the drug, and for example, aromatic hydrocarbons such as toluene, benzene and xylene; esters such as ethyl acetate; halogenated hydrocarbons such as carbon tetrachloride, chloroform and methylene chloride can be used.

In the case of the plaster using a rubber adhesive base, an adhesive base, a softener if necessary and a tackifying agent are mixed while heating by using a blender such as a kneader and a mixer. Subsequently, the drug and the skin irritation suppressant are added and uniformly dispersed to directly spread on the backing, or are spread on the paper or film once subjected to release treatment and then are transferred to the backing by pressure bonding. Next, the surface on the opposite side of the surface contacting the backing in the drug layer is covered with the release liner, and by being cut into the appropriate size, the plaster was obtained. It should be noted that the blending order of each component in the manufacturing method is nothing but one example, and is not limiting.

In a further embodiment, the transdermal preparation is an ointment, a cream, a gel, a gelled cream, a liniment, a lotion, a spray, an aerosol, a reservoir-type patch, etc. other than a cataplasm and a plaster.

The ointment includes not only the drug and the skin irritation suppressant but also, for example, higher fatty acids such as myristic acid or their esters, waxes such as spermaceti, surfactants such as polyoxyethylene, hydrocarbons such as hydrophilic vaseline. Specifically, as one example, the ointment can be manufactured by adding 5-15% by mass of a higher fatty acid or its ester, 1-10% by mass of a surfactant, 0.1-30% by mass of the drug, 0.1-30% by mass of the skin irritation suppressant, 4-10% by mass of waxes and 50-90% by mass of hydrocarbons relative to the total amount, and by mixing them.

The cream comprises, in addition to the drug and the skin irritation suppressant, for example, higher aliphatic acid esters such as myristic acid ester, water, hydrocarbons such as liquid paraffin, emulsifiers such as polyoxyethylene alkyl ethers. Specifically, as one example, the cream can be manufactured by adding 0.1-30% by mass of the drug, 0.1-30% by mass of the skin irritation suppressant relative to the total amount, and an appropriate amount of the higher aliphatic acid ester, water, the hydrocarbons and the emulsifier, and by mixing and stirring them.

The gel comprises, in addition to the drug and the skin irritation suppressant, for example, lower alcohols such as ethanol, water, gelatinizers such as carboxy vinyl polymer, neutralizers such as triethanolamine. Specifically, as one example, the gel can be manufactured by adding 0.5-5% by mass of the gelatinizer to 55% by mass or less of water relative to the total amount of the gel for swelling to obtain a swelled material A, by dissolving 0.1-30% by mass of the drug and 0.1-30% by mass of the skin irritation suppressant in a mixture of 40% by mass or less of glycols and 60% by mass or less of the lower alcohol relative to the total amount of the gel to obtain a lysate B, and by adding the lysate B to the swelled material A and adjusting it at pH 4-7.

The gelled cream has a property intermediate between the gel and the cream, and can be obtained by adding the gelatinizer such as carboxy vinyl polymer and the neutralizer such as diisopropanolamine in addition to components of the cream, and by adjusting it at pH 4-8 or 5-6.5.

The liniment can be obtained by adding 0.1-30% by mass of the drug and 0.1-30% by mass of the skin irritation suppressant to, for example, 10-70 parts by mass of alcohols (monohydric alcohols such as ethanol, propanol, and isopropyl alcohol, and polyhydric alcohols such as polyethylene glycol, propylene glycol and butylene glycol, etc.), 55 parts by mass or less of water, 60 parts by mass or less of fatty acid ester (esters such as adipic acid, sebacic acid and myristic acid), and 10 parts by mass or less of surfactant (such as polyoxyethylene alkyl ether) relative to the total amount.

The lotion comprises, in addition to the drug and the skin irritation suppressant, lower alcohols such as ethanol, water and/or glycols. The lotion can be obtained by adding 0.1-30% by mass of the drug, 0.1-30% by mass of the skin irritation suppressant and an appropriate amount of the lower alcohol, water and/or glycols relative to the total amount, and by mixing and stirring them.

The spray and the aerosol can be manufactured by adding 0.1-30% by mass of the drug and 0.1-30% by mass of the skin irritation suppressant to the known dosage form relative to the total amount.

The reservoir-type patch includes (1) a liner material layer, (2) a drug storing layer, (3) a drug releasing layer and (4) a pressure-sensitive adhesive layer, wherein (2) the drug storing layer is made up of a base which is obtained by adding any of (a) glycols, lower alcohol, water and water-soluble polymer, (b) aliphatic alcohol and polyhydric alcohol and (c) paraffins and silicones.

Pharmaceutically acceptable various additives, for example, a stabilizing agent, an antioxidant, a perfume, a filler, a transdermal absorption enhancer or the like can be added to these transdermal preparations within a range not impairing the object.

Skin irritation may often be caused also by the transdermal absorption enhancer. Skin irritation resulting from this transdermal absorption enhancer, particularly a transdermal absorption enhancer selected from lauric acid diethanolamide (LADA), propylene glycol monolaurate and sorbitan monolaurate may also be reduced.

Further embodiment provides the manufacturing method of the transdermal preparation for reducing skin irritation, which includes the step of adding an effective amount of skin irritation suppressant selected from the group consisting of cholesterol, cholesterol derivatives and cholesterol analogs as well as one or more basic drugs selected from the group consisting of tolterodine, asenapine, bisoprolol, risperidone, nicotine and mitalopram and their pharmaceutically acceptable salts, and/or the pharmaceutical ingredient to be used in a transdermal preparation other than the drug to the transdermal preparation, and provides the method of reducing skin irritation due to a drug and/or pharmaceutical ingredient be used in the transdermal preparation other than the drug.

**Examples**

Hereinafter, the invention will be more specifically explained by showing examples and comparative examples, but is not limited to the following examples.

(Experiment 1)

Changes in production amounts of skin irritation mediators by test substances were examined when cutaneous irritants were caused to act on a human three-dimensional culture epidermal model.

A human three-dimensional epidermal culture model obtained by multilayer-culturing a human normal epidermal cell (LabCyte EPI-MODEL, manufactured by Japan Tissue Engineering Co., Ltd.) was used. LabCyte is a cultured skin cultured in a transwell. For the experiment, LabCyte was used with the transwell.

As test substances, there were used cholesterol (manufactured by Wako Pure Chemical Industries, Ltd.), β-sitosterol (manufactured by TAMA BIOCHEMICAL CO., LTD.), α-spinasterol (manufactured by ChromaDex, Inc.), ergosterol (manufactured by Wako Pure Chemical Industries, Ltd.), campesterol (manufactured by TAMA BIOCHEMICAL CO., LTD.), glycyrrhizin (manufactured by Wako Pure Chemical Industries, Ltd.), glycyrrhetinic acid (manufactured by Wako Pure Chemical Industries, Ltd.), ginsenoside (manufactured by Wako Pure Chemical Industries, Ltd.), squalene (manufactured by Wako Pure Chemical Industries, Ltd.), squalane (manufactured by Tokyo Chemical Industry Co.,Ltd.) and lanosterol (manufactured by ChromaDex, Inc.), which are candidates of a skin irritation suppressant.

As skin irritants, Phorbol 12-Myristate 13-Acetate (PMA, manufactured by Wako Pure Chemical Industries, Ltd.) was used. In addition, as vehicle, olive oil (manufactured by Wako Pure Chemical Industries, Ltd.) was used.

Experiments were conducted by the following group composition.

PMA Only Group: A group with addition of only 0.00001 mol of PMA dispersed in a vehicle, as a test solution.

Test Substance Group: A group with addition of 0.00001 mol of PMA and the test substance which were dispersed in a vehicle, as a test solution.

The experiment was conducted as below. First, LabCyte was put into a 24-well assay plate. Then, 1 ml of assay medium (Japan Tissue Engineering Co., Ltd.) was added so that it was brought into contact with a bottom surface of the cultured skin in each well of the 24-well assay plate, and was pre-cultured in an incubator with 5% of carbon dioxide concentration at 37 °C for 1 hour.

Next, a cell survival rate was measured by the MTT method after causing the test solutions of each group to act. Specifically, 50 μL of the test solution of each group was added to the surface of the LabCyte after preculture, was cultured in the incubator with 5% of carbon dioxide concentration at 37°C for 48 hours, then was transferred to the assay medium comprising 0.5 mg/ml of MTT reagent, and was cultured in the incubator with 5% of carbon oxide level at 37 °C for 3 hours. Then, LabCyte was immersed in 0.3 ml of an isopropanol solution, and the produced blue-purple formazan was extracted for 2 hours. After extraction, an absorbance at 570 nm was measured by a 96 microplate reader. The cell survival rate was calculated as below.

$$\text{Cell survival rate (\%)} = (\text{absorbance of Test Substance Group}) / (\text{absorbance of PMA Only Group}) \times 100$$

Subsequently, a production amount of the skin irritation mediator at a test substance concentration with 80% or more of cell survival rate was measured. Addition concentrations of the test substances are shown in Table 1. As skin irritation mediators, PGE2, IL-1α, IL-6, IL-8 were measured.

The test solutions of each group were added to the cells, the culture solutions incubated for 48 hours were collected, and a production amount of each skin irritation mediator was measured. The skin irritation mediator was measured by using a commercially available ELISA kit (manufactured by R&D Systems, Inc.). A relative production amount of the skin irritation mediator in each test substance was calculated as below, when the production amount of the skin irritation mediator in the PMA Only Group was set to 100.

$$\text{Relative production amount} = (\text{production amount of the mediator in Test Substance Group}) / (\text{production amount of the mediator in PMA Only Group}) \times 100$$

Test results are shown in Table 1. According to Table 1, all of the cholesterol, β-sitosterol, α-spinasterol, ergosterol, campesterol, glycyrrhizin, glycyrrhetinic acid, ginsenoside, squalene, squalane and lanosterol exhibited effects of suppressing the skin irritation mediator production by PMA.

[Table 1]

| Test Substance | Concentration (Mass%) | Relative Production Amount of Skin Irritation Mediator when Production Amount of Skin Irritation Mediator in PMA Only Group is Set to 100 | | | |
|---|---|---|---|---|---|
| | | PGE2 | IL-1$\alpha$ | IL-6 | IL-8 |
| Cholesterol | 1 | 17 | 50 | 0 | 65 |
| $\beta$-sitosterol | 1 | 44 | 101 | 100 | 34 |
| $\alpha$-spinasterol | 0.1 | 43 | 38 | 109 | 72 |
| Ergosterol | 1 | 20 | 71 | 81 | 111 |
| Campesterol | 1 | 38 | 53 | 100 | 106 |
| Glycyrrhizin | 0.01 | 114 | 85 | 105 | 154 |
| Glycyrrhetinic Acid | 0.01 | 48 | 77 | 46 | 81 |
| Ginsenoside | 0.1 | 121 | 77 | 70 | 90 |
| Squalen | 0.01 | 88 | 10 | 0 | 131 |
| Squalane | 0.1 | 41 | 18 | 55 | 39 |
| Lanosterol | 1 | 47 | 76 | 90 | 73 |

(Experiment 2)

<Suppression of production of the skin irritation mediator by cholesterol in a human epidermal cell, -1>

Cholesterol was used as a test substance. The drugs shown in Table 2 were used as the cutaneous irritants.
Tests were conducted by the following group composition.

• Drug Only Group: A group with addition of only the drug dispersed in a vehicle, as a test solution. Concentrations of the drugs are shown in Table 2.

• Drug Plus Cholesterol Group: A group with addition of the drug and 1% by mass of cholesterol which were dispersed in a vehicle, as a test solution. Concentrations of the drugs are shown in Table 2.

The experiment was conducted while other conditions were set in the same manner as in Experiment 1. The results are shown in Table 2. According to Table 2, the cholesterol exhibited effects of suppressing of production of the skin irritation mediator on a wide range of drugs.
[Table 2]

| Drug | Drug Concentration (M) | Relative Production Amount of Skin Irritation Mediator when Production Amount of Skin Irritation Mediator in Drug Only Group is Set to 100 | | | |
|---|---|---|---|---|---|
| | | PGE2 | IL-1$\alpha$ | IL-6 | IL-8 |
| Bisoprolol Fumarate | $1 \times 10^{-3}$ | 87 | 26 | 72 | 73 |
| Risperidone | $1 \times 10^{-3}$ | 80 | 14 | 77 | 38 |
| Nicotine | $2 \times 10^{-3}$ | 58 | 29 | 93 | 67 |
| Tolterodine Tartrate | $8 \times 10^{-5}$ | 58 | 48 | 92 | 66 |

(continued)

| Drug | Drug Concentration (M) | Relative Production Amount of Skin Irritation Mediator when Production Amount of Skin Irritation Mediator in Drug Only Group is Set to 100 | | | |
| --- | --- | --- | --- | --- | --- |
| | | PGE2 | IL-1α | IL-6 | IL-8 |
| (S)-citalopram Oxalate | $1\times10^{-3}$ | 46 | 61 | 85 | 64 |
| Donepezil Hydrochloride | $4\times10^{-4}$ | 33 | 0 | 85 | 59 |

(Experiment 3)

<Suppression of croton oil-induced mouse ear swelling reaction by the test substances>

Suppression of mouse ear swelling was examined through the use of croton oil (Wako Pure Chemical Industries, Ltd.) as a swelling inducer. A 7-week-old ddY female mouse (SPF) was subjected to an experiment. As test substances, cholesterol, glycyrrhizin, glycyrrhetinic acid, squalene, ergosterol, squalane and lanosterol were used. As a vehicle, acetone (Wako Pure Chemical Industries, Ltd.) was used.

Experiments were conducted by the following group composition.

- Positive Control Group: A group which received only the vehicle as a primary test solution, and one hour after, received a 2% croton oil solution as a secondary test solution.
- Test Substance Group: A group in which only the vehicle was administered as a primary test solution, and one hour after, a 2% croton oil solution including 1% by mass of test substance was administered as a secondary test solution.

An inhibition ratio of ear swelling was calculated as below. In this test, a subacute inflammation model is used to examine immediate swelling reactions. In accordance with the group composition, 25 μL of the primary test solution was uniformly coated on back sides of both ears in an etherize mouse, and one hour after, 25 μL of the secondary test solution was uniformly coated in accordance with the group composition. Prior to coating of the secondary test solution, a thickness of auricle was measured by DIAL THICKNESS GAUGE (OZAKI MFG. CO., LTD.). Furthermore, the secondary test solution was administered, and 6 hours after, the auricular thickness was measured in the same way as mentioned above, and an increased amount of the auricular thickness was set by deducting the thickness measured prior to administration of the secondary test solution. The inhibition ratio of ear swelling was calculated as below.

$$\text{Inhibition ratio of ear swelling (\%)} = (\text{increased amount of auricular thickness in Test Substance Group} - \text{increased amount of auricular thickness in Negative Control Group}) / (\text{increased amount of auricular thickness in Positive Control Group} - \text{increased amount of auricular thickness in Negative Control Group}) \times 100$$

The results are shown in Table 3. According to Table 3, it was shown that cholesterol, glycyrrhizin, glycyrrhetinic acid, squalene, ergosterol, squalane and lanosterol suppress croton oil-induced mouse ear swelling reaction.

[Table 3]

| Test Substance | Inhibition Ratio Of Ear Swelling (%) |
| --- | --- |
| Cholesterol | 70.6 |
| Glycyrrhizin | 34.1 |

(continued)

| Test Substance | Inhibition Ratio Of Ear Swelling (%) |
|---|---|
| Glycyrrhetinic Acid | 53.4 |
| Squalene | 44.4 |
| Ergosterol | 38.1 |
| Squalane | 61.6 |
| Lanosterol | 65.9 |

(Experiment 4)

<Examination of reduction in skin irritation in a rabbit by Cholesterol, -1>

Through the use of donepezil hydrochloride as a drug, reduction in skin irritation in a rabbit by cholesterol was examined. A 19-week-old JW female rabbit was subjected to an experiment.

First, transdermal preparations were prepared by the prescription shown in Table 4. A transdermal preparation which contains neither donepezil hydrochloride nor cholesterol was used as Comparative Example 1, and a transdermal preparation which contains only donepezil hydrochloride was used as Comparative Example 2. In Examples 1 to 4, the transdermal preparations which contain donepezil hydrochloride and cholesterol were used. It should be noted that "%" in Table 4 means "percent by mass".

[Table 4]

| | Comparative Example 1 | Comparative Example 2 | Example 1 | Example 2 | Example 3 | Example 4 |
|---|---|---|---|---|---|---|
| Donepezil Hydrochloride | - | 9.0% | 9.0% | 9.0% | 9.0% | 9.0% |
| Cholesterol | - | - | 0.5% | 1.0% | 2.0% | 3.0% |
| SIS Copolymer | 14.7% | 14.2% | 14.1% | 14.0% | 13.9% | 13.8% |
| Polyisobutylene | 6.0% | 6.0% | 6.0% | 6.0% | 5.9% | 5.8% |
| Liquid Paraffin | 41.0% | 36.5% | 36.3% | 36.1% | 35.7% | 35.3% |
| Alicyclic Saturated Hydrocarbon Resin | 38.3% | 34.3% | 34.1% | 33.9% | 33.5% | 33.1% |

Specifically, donepezil hydrochloride, cholesterol, liquid paraffin and toluene are mixed, which was mixed with solutions of a styrene-isoprene-styrene (SIS) block copolymer, an alicyclic saturated hydrocarbon resin, a polyisobutylene and toluene which were separately prepared, and a mixture was obtained. This mixture was spread on a polyethylene terephthalate film subjected to mold release treatment, and its solvent was dried for removal, and a drug layer was formed, on which the backing was laminated. Then, after being cut, the transdermal preparation was obtained.

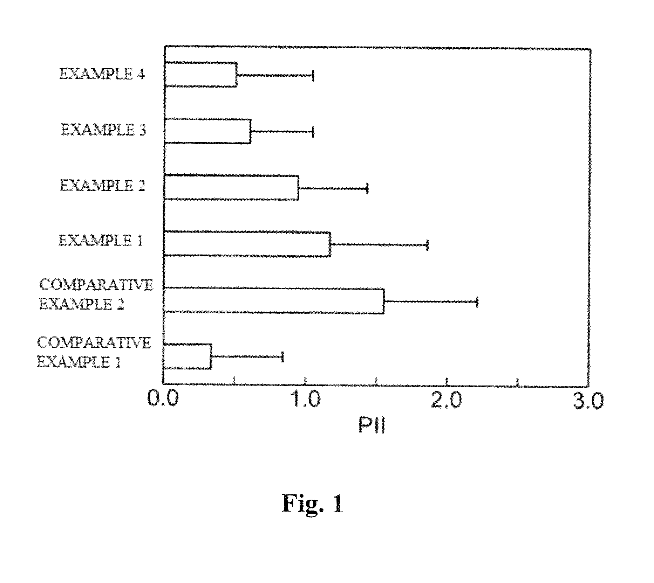

Subsequently, the transdermal preparation of each group was applied to a shaved back of the rabbit a single time for 24 hours. Erythema and swelling on the applied site were evaluated 1, 24 and 48 hours after peel-off of the transdermal preparation in accordance with the judgment standard of Draize et al. and an average value of the skin primary irritation index (PII) was calculated. The results are shown in Fig. 1. According to Fig. 1, the transdermal preparations in the groups which contain 0.5% cholesterol (Example 1), 1% cholesterol (Example 2), 2% cholesterol (Example 3) and 3% cholesterol (Example 4) exhibited reduced PII depending on the dose of cholesterol, in comparison with the transdermal preparation containing no cholesterol (Comparative example 2).

(Experiment 5)

< Skin permeability test of the drug in a hairless mouse >

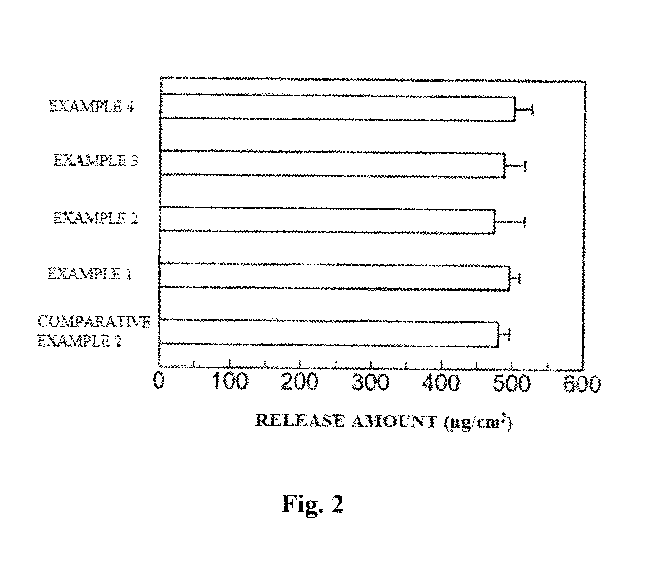

The skin of a hairless mouse was peeled off from the side of the body, the transdermal preparation (about 3 cm$^2$) in Examples 1-4 and Comparative examples in Experiment 4 was applied to the horny layer side. Then, its dermis side was faced to a receptor side and was set on a flow-through cell in which warm water was circulated around the outer circumference. Through the use of a PBS in the receptor layer, sampling was carried out at a flow rate of about 5 mL/hr every 3 hours for 24 hours. A flow volume of the resulting receptor solution was accurately measured, a drug concentration was measured by high-performance liquid chromatography, and then an amount of release was calculated. The results are shown in Fig. 2. According to Fig. 2, the transdermal preparations in the cholesterol groups which contain no cholesterol (Comparative example 2), 0.5% cholesterol (Example 1), 1% cholesterol (Example 2), 2% cholesterol (Example 3) and 3% cholesterol (Example 4) exhibited an equal amount of donepezil release.
It was confirmed from the results of Experiments 4 and 5 that the cholesterol reduces skin irritation due to the drug without preventing the release of donepezil.

(Experiment 6)

<Suppression of the skin irritation mediator production by cholesterol in a human epidermal cell, -2>

Cholesterol was used as a test substance. Asenapine and lauric acid diethanolamide (LADA) were used as the cutaneous irritants.
Tests were conducted by the following group composition.

• Solvent Group: A group including only vehicle. Olive oil was used as a vehicle.
• skin Irritant Only Group: A group with addition of only cutaneous irritant dispersed in a vehicle, as a test solution. The concentration of asenapine maleate to be added was 0.6% (asenapine group), and that of LADA was 0.03% (LADA group).
• Skin Irritant Plus Cholesterol Group: A group with addition of the drug and 1% by mass of cholesterol which were dispersed in a vehicle, as a test solution.

The experiment was conducted while other conditions were set in the same manner as Experiment 1. The results are shown in Tables 5 and 6. The cholesterol also exhibited effects of suppressing the skin irritation mediator production on skin irritation due to asenapine and LADA.
[Table 5]

| Skin Irritation Substance | Production Amount of Skin Irritation Mediator (pg/ml) | | | | |
|---|---|---|---|---|---|
| | PGE2 | IL-1A | IL-6 | IL-8 | TNFA |
| Solvent Group | 86.63 | 12.24 | 0.00 | 84.07 | 0.00 |
| Asenapine Group | 125.85 | 17.24 | 0.00 | 174.90 | 0.00 |
| Asenapine + Cholesterol Group | 89.06 | 11.87 | 0.00 | 100.59 | 0.00 |

[Table 6]

| Skin Irritation Substance | Production Amount of Skin Irritation Mediator (pg/ml) | | | | |
|---|---|---|---|---|---|
| | PGE2 | IL-1$\alpha$ | IL-6 | IL-8 | TNF$\alpha$ |
| Solvent Group | 21.71 | 18.42 | 0.00 | 111.41 | 0.00 |
| LADA Group | 30.64 | 29.85 | 0.00 | 160.29 | 0.00 |
| LADA+ Cholesterol Group | 20.39 | 26.35 | 0.00 | 93.01 | 0.00 |

(Experiment 7)

<Examination of reduction in the skin irritation in a rabbit by cholesterol, -2>

Through the use of donepezil hydrochloride, tolterodine and asenapine maleate as drugs, reduction in the skin irritation in a rabbit by cholesterol was examined. The experimental method was the same as that in Experiment 4.

first, transdermal preparations were prepared. As to donepezil chloride, Comparative Example 2 (transdermal preparation including only donepezil hydrochloride) and Example 4 (transdermal preparation including donepezil hydrochloride and cholesterol) shown in Table 4 were used. As to the tolterodine and asenapine, the transdermal preparation was prepared by the prescription shown in Table 7. It should be noted that "%" in Table 7 means "percent by mass".

[Table 7]

|  | Comparative Example 3 | Example 5 | Comparative Example 4 | Example 6 |
|---|---|---|---|---|
| Tolterodine | 25.0% | 25.0% | - | - |
| Asenapine Maleate | - | - | 12.0% | 12.0% |
| Cholesterol | - | 5.0% | - | 3.0% |
| Polyvinylpyrrolidone | 5.0% | 5.0% | - | - |
| Duro Tak 4287 | 70.0% | 65.0% | - | - |
| SIS Copolymer | - | - | 11.9% | 11.4% |
| Liquid Paraffin | - | - | 36.5% | 35.6% |
| Alicyclic Saturated Hy drocarbon Resin | - | - | 39.6% | 38.0% |

The preparative method of the transdermal preparation was a general method. Specifically, in Comparative Example 3 and Example 5, the mixing of the materials described in Table 7 resulted in preparing a coating liquid. The coating liquid was spread on a polyethylene terephthalate film subjected to mold release treatment, the solvent was dried for removal, and the drug layer was formed, on which the backing was laminated. Then, after being cut, the transdermal preparation was obtained. In Comparative Example 4 and Example 6, asenapine maleate, cholesterol, liquid paraffin and toluene are mixed, which was mixed with solutions of a stylene-isoprene-stylene (SIS) block copolymer, an alicyclic saturated hydrocarbon resin and toluene which were separately prepared, and a mixture was obtained. This mixture was spread on a polyethylene terephthalate film subjected to mold release treatment, and its solvent was dried for removal, and a drug layer was formed, on which the backing was laminated. Then, after being cut, the transdermal preparation was obtained.

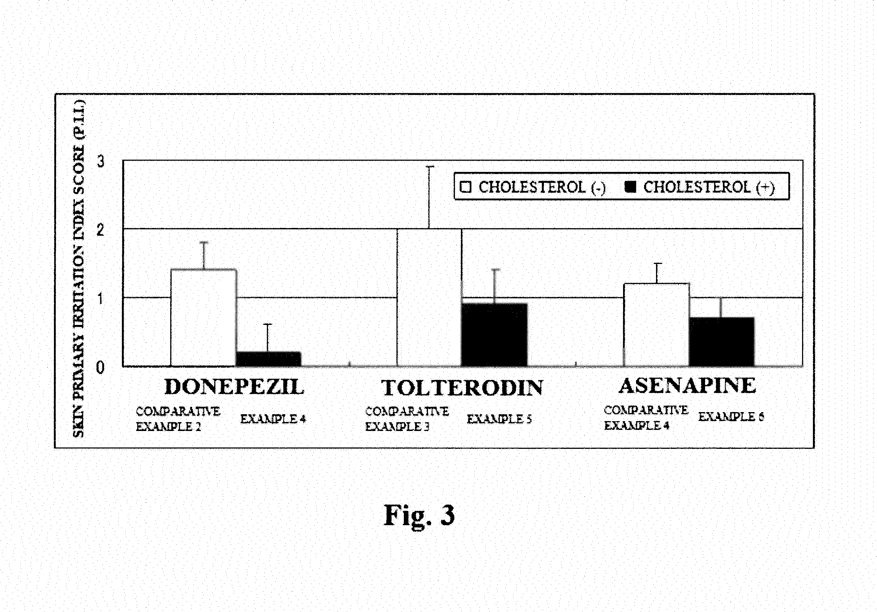

Next, the transdermal preparation of each group was applied to a shaved back of the rabbit a single time for 24 hours. Erythema and swelling on the applied site were evaluated 1, 24 and 48 hours after peel-off of the transdermal preparation in accordance with the judgment standard of Draize et al. and an average value of the skin primary irritation index (PII) was calculated based on each score. The results are shown in Fig. 3. According to Fig. 3, it was found that the groups containing cholesterol (Example 4, 5 and 6) exhibited reduction in skin irritation due to the drug, in comparison with the groups containing no cholesterol (Comparative examples 2, 3 and 4).

**Industrial Applicability**

The skin irritation suppressant for the transdermal preparation having sufficient reduction effect of skin irritation on drugs, and the transdermal preparation comprising the skin irritation suppressant can be provided.

**Claims**

1. A skin irritation suppressant for suppressing the skin irritation due to a drug and a pharmaceutical ingredient to be used in a transdermal preparation other than the drug, comprising a sterol compound selected from the group consisting of cholesterol, cholesterol derivatives and cholesterol analogs, wherein the drug is one or more basic drugs selected from the group consisting of tolterodine, asenapine, bisoprolol, risperidone, nicotine and mitalopram,

and their pharmaceutically acceptable salts.

2. The transdermal preparation according to claim 1, wherein the pharmaceutical ingredient to be used in a transdermal preparation other than the drug, is a transdermal absorption enhancer.

3. The transdermal preparation according to claim 3, wherein the transdermal absorption enhancer is selected from lauric acid diethanolamide, propylene glycol monolaurate and sorbitan monolaurate.

4. A transdermal preparation, comprising an effective amount of the skin irritation suppressant according to any one of claims 1 to 3, and the drug and/or the pharmaceutical ingredient to be used in a transdermal preparation other than the drug.

5. The transdermal preparation according to claim 4, which comprises 0.1-30% by mass of the skin irritation suppressant relative to a total amount of the transdermal preparation.

6. The transdermal preparation according to claim 4 or 5, wherein the mass ratio of the drug to the skin irritation suppressant is from 30:1 to 1: 10 if the drug is comprised.

**Fig. 1**

**Fig. 2**

Fig. 3

<table>
<tr><td colspan="2" align="center">**INTERNATIONAL SEARCH REPORT**</td><td>International application No.<br>PCT/JP2011/060290</td></tr>
</table>

A. CLASSIFICATION OF SUBJECT MATTER
*A61K47/10*(2006.01)i, *A61K9/70*(2006.01)i, *A61K31/137*(2006.01)i, *A61K31/138*
(2006.01)i, *A61K31/343*(2006.01)i, *A61K31/407*(2006.01)i, *A61K31/465*
(2006.01)i, *A61K31/517*(2006.01)i
According to International Patent Classification (IPC) or to both national classification and IPC

B. FIELDS SEARCHED

Minimum documentation searched (classification system followed by classification symbols)
A61K47/10, A61K9/70, A61K31/137, A61K31/138, A61K31/343, A61K31/407,
A61K31/465, A61K31/517

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | WO 2008/44336 A1 (Hisamitsu Pharmaceutical Co., Inc.), 17 April 2008 (17.04.2008), claims; paragraphs [0016], [0023]; examples (Family: none) | 1-6 |
| Y | WO 2006/82888 A1 (Kyorin Pharmaceutical Co., Ltd.), 10 August 2006 (10.08.2006), claims; paragraphs [0012], [0020] to [0021], [0032] & EP 1844773 A1 & KR 10-2007-0100834 A | 1-6 |

| ☒ Further documents are listed in the continuation of Box C. | ☐ See patent family annex. |
|---|---|

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>09 June, 2011 (09.06.11) | Date of mailing of the international search report<br>21 June, 2011 (21.06.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| | International application No. |
|---|---|
| | PCT/JP2011/060290 |

C (Continuation).   DOCUMENTS CONSIDERED TO BE RELEVANT

| Category* | Citation of document, with indication, where appropriate, of the relevant passages | Relevant to claim No. |
|---|---|---|
| Y | YASUKAWA, K. et al, Inhibitory Effects of Sterols Isolated from Chlorella vulgaris on 12-O-Tetradecanoylphorbol-13-acetate-Induced Inflammation and Tumor Promotion in Mouse Skin., Biol Pharm Bull, 1996, Vol.19, No.4, p.573-576 | 1-6 |
| Y | JP 2002-265346 A  (Pola Chemical Industries Inc.), 18 September 2002 (18.09.2002), claims; paragraphs [0006] to [0007]; examples (Family: none) | 1-6 |
| Y | Ichiro KATAYAMA et al., "Jusho Atopic Dermatitis no Nanjika Kijo o Fumaeta Chiryoho no Kakuritsu ni Kansuru Kenkyu Lipid Raft o Hyoteki to shita Atarashii Allergic Disease Chiryo Senryaku no Kento", Jusho Atopic Dermatitis no Nanjika Kijo o Fumaeta Chiryoho no Kakuritsu ni Kansuru Kenkyu Heisei 15 to 17 Nendo Sogo Kenkyu Hokokusho, 2006, pages 63 to 68 | 1-6 |
| Y | KASAHARA, Y. et al, Carthami Flos extract and its component, stigmasterol, inhibit tumor promotion in mouse skin two-stage carcinogenesis, Phytotherapy Research, 1994, Vol.8, No.6, p.327-31 | 1-6 |
| Y | KAMINAGA, T. et al, Inhibitory effect of Poria cocos on 12-O-tetradecanoylphorbol-13-acetate-induced ear edema and tumor promotion in mouse skin, Phytotherapy Research, 1996, Vol.10, No.7, p.581-584 | 1-6 |

Form PCT/ISA/210 (continuation of second sheet) (July 2009)

**EP 2 564 873 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007284378 A
- JP 2007045738 A
- JP 4501415 A
- WO 2009075258 A

**Non-patent literature cited in the description**

- **DRAIZE JH et al.** *J Pharmacol Exp Ther.,* 1944, vol. 82, 377-390